# EUROPEAN PATENT APPLICATION

(11) **EP 4 241 773 A1**
(43) Date of publication of application: **13.09.2023**
(21) Application number: 21889548.0
(22) Date of filing: 03.11.2021
(51) Int. Cl.: A61K 31/506, A61K 45/06, A61P 35/02, C12Q 1/6886

(54) **PHARMACEUTICAL COMPOSITION FOR TREATING LEUKEMIA COMPRISING FLT3 INHIBITOR**

(30) Priority: 05.11.2020 KR 20200147156
(71) Applicant: Hanmi Pharm. Co., Ltd., Hwaseong-si, Gyeonggi-do 18536 (KR)
(72) Inventor: BAE, In Hwan, Hwaseong-si, Gyeonggi-do 18469 (KR); SONG, Ji Young, Hwaseong-si, Gyeonggi-do 18469 (KR); CHOI, Jae Yul, Hwaseong-si, Gyeonggi-do 18469 (KR); AHN, Young Gil, Hwaseong-si, Gyeonggi-do 18469 (KR)
(74) Representative: Cooley (UK) LLP
(86) International application number: PCT/KR2021/015794
(87) International publication number: WO 2022/098083

(57) **Abstract**

[Summary]

The present invention relates to a pharmaceutical composition for the treatment of leukemia comprising an FMS-like tyrosine kinase-3 (FLT3) inhibitor and a pharmaceutically acceptable excipient.

## Description

### [Technical Field]

The present invention relates to a pharmaceutical composition for the treatment of leukemia comprising an FMS-like tyrosine kinase-3 (FLT3) inhibitor and a pharmaceutically acceptable excipient.

### [Background Art of the Invention]

Kinase serves as a medium for the reaction moving high energy molecules, especially phosphate group of ATP, to substrate. Kinase plays a role of speeding up the reaction by stabilizing anhydrous phosphate bond and placing substrate and phosphate group at specific positions. The transition state that results from interacting with a negatively charged phosphate group is in most cases electrostatically stabilized through a positively charged surrounding amino acid, and some kinases also use metal cofactors to coordinationally bind to the phosphate group.

Kinases can be divided into various groups such as protein kinases, lipid kinases, and carbohydrate kinases. Depending on the state of phosphorylation, proteins, lipids or carbohydrates can change their activity, reactivity and ability to bind to other molecules. Kinases have broad effects on intracellular signal transduction and control the complicated biological mechanism within the cells. Through phosphorylation, the activity of some molecules is either strengthened or inhibited, and their ability to interact with other molecules can be adjusted. Because a large number of kinases react depending on environmental conditions or signals, cells can control molecules within the cells through kinases depending on the situation. Therefore, kinases play a very important role in the growth, differentiation, proliferation, survival, metabolism, signal transduction, cellular transport and secretion of the cells and other numerous cellular reaction routes.

Kinases are found various species ranging from bacteria to mold, insects and mammals, and more than 500 kinases have been found in humans so far.

Protein kinases either increase or decrease the activity of protein, stabilizes it or becomes the marker for degradation, place it in a specific cell compartment, or start or disturb its interaction with other proteins. Protein kinases are known to account for most of all kinases and have been a subject of major researches. Protein kinases play a role in controlling protein and enzyme as well as cell signal transduction together with phosphatase, and cell proteins are the subject of numerous covalent bonds, but since there aren't many reversible covalent bonds like phosphorylation, the phosphorylation of protein can be described as having a regulatory function. Protein kinases often have a number of substrates, and sometimes, specific proteins can act as the substrate in one or more kinases. For this reason, a protein kinase is named using the factor controlling its own activity. For example, calmodulin-dependent protein kinases are controlled by calmodulin. Sometimes, kinases are also divided into subgroups. For example, type 1 and type 2 cyclic AMP-dependent protein kinases are composed of the same enzyme subunit, but they are controlled as other control subunits are bonded to cyclic AMP.

Protein kinase, which is an enzyme which catalyzes the phosphorylation of hydroxy group located in the tyrosine, serine, and threonine residues of protein, plays an important role in the transduction of growth factor signal which induces the growth, differentiation and proliferation of cells (Melnikova, I. et al., Nature Reviews Drug Discovery, 3(2004), 993), and it has been reported that abnormal expression or mutation of specific kinases are frequent in cancer cells.

In general, one of the methods by which cells recognize external stimuli is through tyrosine kinases, which are receptors on the cell membrane. Receptor tyrosine kinase (RTK) is composed of extracellular part exposed out of a cell, intracellular part exposed to the cytoplasm within a cell and membrane passing part which passes through the plasmalemma located in the middle. The extracellular part of the receptor is a part with which a specific ligand is combined, and the intracellular part performs a function transferring the activation signal of the receptor activated by the ligand into the cell. Since receptor tyrosine kinases have a domain with tyrosine kinase activity on the C-terminal region exposed within the cell, the attachment of a specific ligand to the extracellular part activates the kinase enzyme of the tyrosine kinase domain of the C-terminal exposed to the cytoplasm part of the receptor protein, which phosphorylates the tyrosine at each other's C-terminal in a dimer. This process of tyrosine phosphorylation becomes the most important process in which signals about extracellular stimuli are delivered into the cell. There are a number of receptors known to have tyrosine kinase activity delivering extracellular stimuli into the cell with such a mechanism. Typical examples include FLT3, VEGFR and SYK.

Among them, FMS-like tyrosine kinase 3 (FLT3), which is a receptor tyrosine kinase, is usually expressed in haemopoietic precursor cells by hematopoietic blast, and it plays an important role in the expression and immune system of typical stell cells. Abnormal overexpression and mutation of FLT3 are oftentimes observed in patients with leukemia. Especially, various mutations such as D835V, D835Y and ITD (internal tandem duplication) of FLT3 are observed in acute myelogenous leukemia (AML). Acute myelogenous leukemia is a simplex hematopoietic stem cell disease characterized by abnormal growth and differentiation of blasts in bone marrow and peripheral blood. FLT3 has recently been considered one of the most important targets from an AML therapeutic standpoint.

In adults' AML, the mutations of RAS and p53 genes are reported in about 20% and 5% of adults' AML, whereas the mutations of FLT3 genes are found in about 30% of adults' AML. The most typical problem in AML is that a mutation of FLT3 which causes a bad prognosis is activated. FLT3 mutations are divided largely into two types. One is the internal tandem duplication (ITD) within the juxtamembrane region, and the other is the point mutation within the tyrosine kinase domain (TKD). FLT3-ITD, which is a mutation found most often, is activated in about 23% of early stage AML, patients. A patient with ITD mutation shows a bad prognosis and a high recurrence rate. Another major FLT3 mutation is FLT3 TKD mutation, which accounts for about 7% of early stage AML cases. In the residues of aspartate 835 (D835) substituted by various amino acids, point mutations occur less often than ITD mutations, but they are one of the most common mutations. In addition, another major activation method of FLT3 in AML is the overexpression of wild type FLT3 protein.

As described above, the activation of ITD (internal tandem duplication) mutation in FLT3 is detected in about 20% of patients with acute myelogenous leukemia, and this is related to bad prognosis. It has been found through researches that FLT3-ITD is a driver lesion playing a role in causing the onset of malignant disease, and it can be an valid therapeutic target in human AML (Non-patent literature 1). Mutation of FLT3 gene is a phenomenon occurring frequently in AML, and it is usually accompanied by ITD (internal tandem duplication) in the juxtamembrane domain coding region or point mutation of tyrosine kinase domain (TKD).

Both FLT3-ITD mutation and FLT3-TKD mutation cause ligand-independent growth due to the dimerization of all components and the activation of FLT3 receptors. The higher mutation rate compared to the wild type allelic gene of FLT3-ITD is related to bad prognosis in both adults and children (Non-patent literature 2). Other types of leukemia, such as chronic myelomonocytic leukemia (CMML), can also have activating mutations of FLT3. Therefore, FLT3 having activating mutations is an important target for various types of cancer (Non-patent literature 3 and Non-patent literature 4).

Especially, various TKD variants have been reported as various acquired resistance in the analysis of FLT3 inhibitors approved already or under development for the patients with refractory or recurrent acute myelogenous leukemia. Typical examples include K429A variant of receptor domain (RD), Y572C of JM domain, A627P, N676K/D/I/S and F691L of TK1 domain, and D835Y/F/I/H/V/A and Y842C/H/N of TK2 domain (Non-patent literature 5). In the analysis of resistant patients in the clinical studies on FLT3 inhibitors, the most important acquired resistance includes F691L and in the case of F691L, it is a gatekeeper variant which can inhibit the activity of the drug. Therefore, the development of a drug which can target F691L alone or double mutant of ITD-F691L is considered as an unsatisfied medical need (Non-patent literature 6).

Spleen tyrosine kinase (SYK), primarily expressed in blood cells, plays an important role as a signal transduction route of other immune receptors such as B-cell receptors and mast cells. Spleen tyrosine kinase is also expressed in nonhematopoietic cells such as nerve cells and vascular endothelial cells. It has been proved in a recent research that it plays an important role in the oxidation of various cell stimulation including IL-1, TNF-α and ITGB1. SYK is known as potentially a good target in various hematological malignant tumors, autoimmune diseases and other inflammatory responses (Non-patent literature 7 and Non-patent literature 8).

### [Prior technology literature]

### [Non-patent literature]

Catherine et al., Nature, 2012, 485, 260 - 263
A S Moore et al., Leukemia, 2012, 26, 1462 - 1470
Cancer Cell, (2007), 12: 367 - 380
Current Pharmaceutical Design (2005), 11, 3449 - 3457
H Kiyoi et al., Cancer Science (2020), 111, 312
Christine et al., Cancer Discovery, 9, (2019), 1050
Liu et al., Journal of Hematology & Oncology (2017), 10, 145
Yamada T et al., J. Immunol., (2001) 167, 283 - 288

### [Description of invention]

### [Problems to be solved by the invention]

One aspect of the present invention is to provide a pharmaceutical composition for the treatment of leukemia comprising an FMS-like tyrosine kinase-3 (FLT3) inhibitor and a pharmaceutically acceptable excipient.

Another aspect of the present invention is to provide a medical use to treat a cancer carrying F691L acquired mutation in accordance with the above one aspect.

### [Means to resolve the problems]

One aspect of the present invention provides a pharmaceutical composition for the treatment of leukemia comprising an FMS-like tyrosine kinase-3 (FLT3) inhibitor and a pharmaceutically acceptable excipient.

Another aspect of the present invention provides a medical use to treat a cancer carrying F691L acquired mutation in accordance with the above one aspect.

One embodiment provides a pharmaceutical composition comprising an FMS-like tyrosine kinase-3 (FLT3) inhibitor and a pharmaceutically acceptable excipient, wherein
the above FLT3 inhibitor is at least one compound selected from a group comprising a compound of the chemical formula 1 below, its stereoisomer, its tautomer, and their combination; or
is a pharmaceutically acceptable salt of this selected, at least one compound or a solvate of this selected, at least one compound; and
the said pharmaceutical composition is a pharmaceutical composition to treat leukemia, and the said leukemia cancer cells have internal tandem duplication (ITD) variation within FLT3 genes, and
the pharmaceutical composition further includes at least one FLT3 mutation selected among F691L, D835Y, D835F, D835I, D835H, D835V and D835A.

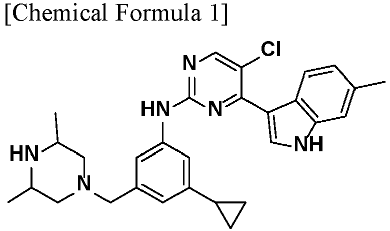

In one embodiment, the said leukemia is acute myeloid leukemia (AML).

In one embodiment, the said pharmaceutical composition can be coadministered with an SYK inhibitor simultaneously or sequentially.

In this specification, the term "simultaneously" refers to the administration of two or more components practically simultaneously or simultaneously through the same route, and when administered practically simultaneously, the route of administration can be either the same or different. In this specification, the term "sequentially" refers to the administration of two or more components at different points in time, and the route of administration can be either the same or different.

Other embodiments of pharmaceutical composition containing FLT3 inhibitor are as follows:
(1) It can be a pharmaceutical composition for the treatment of cancer containing a compound selected among compound of the Chemical Formula A below, its stereoisomer, tautomer, solvate and pharmaceutically acceptable salt as active ingredient. 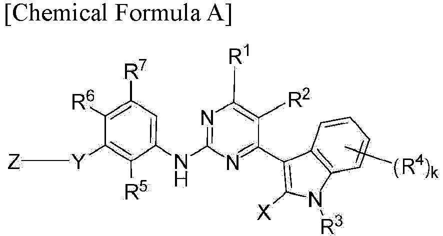 In the above Chemical Formula A,
   R¹ is hydrogen, halogen, hydroxy, C₁₋₄ alkoxy or -NRₐR_{b},
   wherein Rₐ and R_{b} are independently hydrogen or C_{1-4 alkyl}, respectively;
   R² is hydrogen, halogen, cyano, nitro, amino, carboxamide, formyl, halo C₁₋₄ alkyl or C₁₋₄ alkyl;
   R³ is hydrogen, halogen, hydroxy, halo C₁₋₄ alkyl, C₁₋₄ alkyl, C₂₋₄ alkenyl or C₂₋₄ alkynyl;
   each R⁴, mutually independently, is hydrogen, halogen, hydroxy, cyano, nitro, amino, -S (=O)₁-R_{c}-, halo C₁₋₄ alkyl, C₁₋₄ alkoxy, hydroxy C₁₋₄ alkyl, C₁₋₄ alkyl, C₂₋₄ alkenyl, C₂₋₄ alkynyl, -NR_{d}Rₑ, -CO₂Rₑ or -CO-NR_{d}Rₑ,
   wherein R_{c} is C₁₋₄ alkyl or -NR_{d}Rₑ;
   R_{d} and Rₑ, mutually independently, is hydrogen or C₁₋₄ alkyl,
   1 is an integer from 0 to 2;
   k is an integer from 0 to 4;
   R⁵ and R⁶, mutually independently, is hydrogen, halogen, hydroxy, nitro, amino, C₁₋₄ alkoxy or C₁₋₄ alkyl;
   R⁷ is hydroxy C₁₋₄ alkyl, C₁₋₄ alkyl, C₂₋₄ alkenyl, C₂₋₄ alkynyl, C₃₋₇ cycloalkyl or C₃₋₉ heterocycloalkyl,
   wherein C₃₋₇ cycloalkyl or C₃₋₉ heterocycloalkyl can be substituted or unsubstituted for halogen, C₁₋₄ alkyl or halo C₁₋₄ alkyl;
   X is H or OH;
   if X is OH, the compound of the chemical formula A may include the tautomer structure as shown in the following chemical formula B: 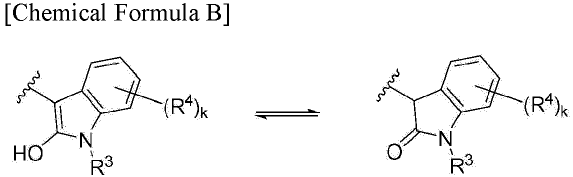 In the chemical formula B, the definition of R³, R⁴, and k is the same as in the chemical formula A;
      Y is -(CH₂)ₘ-, -(CH₂)ₘ-O-(CH₂)ₙ-, -(CH₂)ₘ-CO-(CH₂)ₙ-, -(CH₂)ₘ-NR⁸-(CH₂)ₙ- or -(CH₂)ₘ-SO₂-(CH₂)ₙ-,
      wherein R⁸ is hydrogen or C₁₋₄ alkyl;
      each of m and n is an integer from 0 to 2 independently from each other;
      Z is in the structure of the following chemical formula C; 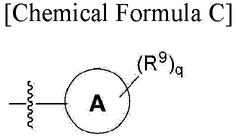 In the above Chemical Formula C, 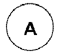 is C₃₋₁₀ cycloalkyl or C₂₋₁₁ heterocycloalkyl;
         R⁹ is halogen, hydroxy, cyano, nitro, amino, thiol, formyl, halo C₁₋₄alkyl, C₁₋₄ alkoxy, linear or branched hydroxy C₁₋₄ alkyl, linear or branched C₁₋₄ alkyl, C₂₋₄ alkenyl, C₂₋₄ alkynyl, C₃₋₁₀ cycloalkyl, C₂₋₉ heterocycloalkyl, hydroxy C₂₋₉ heterocycloalkyl, linear or branched hydroxy C₁₋₄ alkylcarbonyl, -NR¹⁰R¹¹, -COR¹², -COOR¹² or -SO₂R¹³;
         q is an integer from 0 to 5; however, if 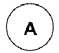 is piperazinyl or piperidinyl, q is not 0;
         wherein 2 or more R⁹ can be connected with each other or fused with 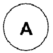 to form bicycloalkyl, heterobicycloalkyl, spirocycloalkyl, or spiroheterocycloalkyl) of 7 to 12 circles;
         each of R¹⁰ and R¹¹ is hydrogen, hydroxy C₁₋₄ alkyl, halo C₁₋₄ alkyl, C₁₋₄ alkyl, C₂₋₄ alkenyl or C₂₋₄ alkynyl, independently from each other;
         R¹² is hydrogen, hydroxy, hydroxy C₁₋₄ alkyl, halo C₁₋₄ alkyl, C₁₋₄ alkyl, C₂₋₄ alkenyl, C₂₋₄ alkynyl, C₃₋₁₀ cycloalkyl or C₂₋₉ heterocycloalkyl;
         R¹³ is hydroxy, halo C₁₋₄ alkyl, C₁₋₄ alkyl, C₂₋₄ alkenyl, C₂₋₄ alkynyl, C₃₋₁₀ cycloalkyl, C₂₋₉ heterocycloalkyl, aryl or -NR_{f}R_{g};
         each of R_{f} and R_{g} is hydrogen or C₁₋₄ alkyl, independently from each other.
(2) In the above section (1), It can contain a compound selected among the compound of the Chemical Formula D below, its stereoisomer, tautomer, solvate and pharmaceutically acceptable salt as active ingredient. 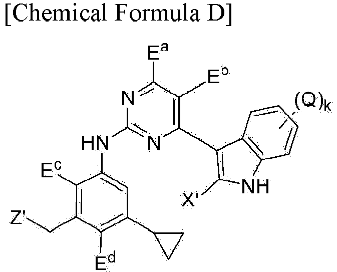 In the above Chemical Formula D,
   E^{a} is hydrogen, hydroxy or C₁₋₄ alkoxy;
   E^{b} is hydrogen, halogen, C₁₋₄ alkyl or C₁₋₄ fluoroalkyl;
   E^{c} and E^{d} are hydrogen or hydroxy, independently from each other;
   X' is hydrogen or hydroxy;
   k is an integer from 0 to 4;
   each Q is hydroxy, halogen, C₁₋₄ alkyl, hydroxy C₁₋₄ alkyl or C₁₋₄ alkoxy, independently from each other;
   Z' is a monovalent functional group shown in the chemical formula E; 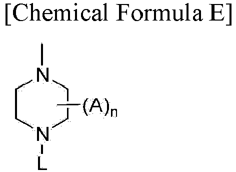
   At this time, in the above chemical formula E, n is an integer from 1 to 8;
   Each substituent A is a functional group selected among hydroxy, C₁₋₄ alkyl and hydroxy C₁₋₄ alkyl, independently from each other, wherein if n is 2 or above, Z' can form a bridged heterobicycloalkyl ring of 7 to 12 circles as two As out of those n's are connected with each other to form an alkylene bridge, or it can form a spiroheterocycloalkyl ring of 7 to 12 circles as two As are spiro connected;
   L is hydrogen, C₁₋₄ alkyl, hydroxy or hydroxyC₁₋₄ alkyl.
(3) In the above section (2), a pharmaceutical composition in which E^{b} is halogen, n is 2, and A includes methylin compound.
(4) In the above section (2), a pharmaceutical composition in which Z' includes 3,5-dimethylpiperazine-1-yl compound.
(5) In the above section (2), a pharmaceutical composition in which E^{b} includes chlorine or fluorine compound.
(6) In the above section (1) or (2), a pharmaceutical composition in which the compound of the above chemical formula A includes a compound selected from the group composed of the following compounds:
   1) 5-chloro-*N-*(3-cyclopropyl-5-(((3*R*, *5S*)-3,5-dimethylpiperazine-1-yl)methyl) phenyl)-4-(6-fluoro-1*H*-indol-3-yl)pyrimidine-2-amin
   2) 5-chloro-4-(6-chloro-1*H*-Indol-3-yl)-*N-*(3-cyclopropyl-5-(((3*R*, 5*S*)-3,5-dimethylpiperazine-1-yl) methyl) phenyl)pyrimidine-2-amin
   3) 2-((2*R*, 6*S*)-4-(3-((5-chloro-4-(6-fluoro-1*H*-indol-3-yl)pyrimidine-2-yl)amino)-5-cyclopropylbenzyl)-2,6 -dimethylpiperazine-1-yl)ethane-1-ol
   4) 2-((2*R*, 6*S*)-4-(3-((5-chloro-4-(1*H*-indol-3-yl)pyrimidine-2-yl)amino)-5-cyclopropylbenzyl)-2,6-dimethy lpiperazine-1-yl)ethane-1-ol
   5) 2-((2*R*, 6*S*)-4-(3-((5-chloro-4-(6-methyl-1*H*-indol-3-yl)pyrimidine-2-yl)amino)-5-cyclopropylbenzyl)-2, 6-dimethylpiperazine-1-yl)ethane-1-ol
   6) (*R*)-5-chloro-*N*-(3-cyclopropyl-5-((3-methylpiperazine-1-yl)methyl)penyl)-4-(1*H*-indol-3-yl)pyri midine-2-amin
   7) (*R*)-5-chloro-*N*-(3-cyclopropyl-5-((3-methylpiperazine-1-yl)methyl)penyl)-4-(6-methyl-1*H*-indol -3-yl)pyrimidine-2-amin
   8) 5-chloro-*N*-(3-cyclopropyl-5 -(((3*R*,5*S*)-3,5 -dimethylpiperazine-1-yl)methyl) phenyl)-4-(6-methyl-1*H*-indol-3-yl)pyrimidine-2-amin
   9) 5-chloro-*N*-(3-cyclopropyl-5-(((3*S*,*5R*)-3-ethyl-5-dimethylpiperazine-1-yl)methyl) phenyl)-4-(6-methyl-1*H*-indol-3-yl)pyrimidine-2-amin
   10) 5-chloro-*N*-(3-cyclopropyl-5-((3,5-dimethylpiperazine-1-yl)methyl) phenyl)-4-(6-methyl-1*H*-indol-3-yl)pyrimidine-2-amin
   11) *N*-(3-cyclopropyl-5-(((*3R*,*5S*)-3,5-dimethylpiperazine-1-yl) methyl)phenyl)-4-(6-methyl-1*H*-indol-3-yl)pyrimidine-2-amin
   12) *N*-(3-cyclopropyl-5-(((3*R*,5*S*)-3,5-dimethylpiperazine-1-yl) methyl)phenyl)-5-fluoro-4-(6-methyl-1*H*-indol-3-yl)pyrimidine-2-amin
   13) *N*-(3-cyclopropyl-5-(((3*R*,5*S*)-3,5-dimethylpiperazine-1-yl) methyl)phenyl)-4-(1*H*-indol-3-yl)-5-methylpyrimidine-2-amin
   14) *N*-(3-cyclopropyl-5-(((3*R*,5*S*)-3,5-dimethylpiperazine-1-yl) methyl)phenyl)-5-methyl-4-(6-methyl-1*H*-indol-3-yl)pyrimidine-2-amin
   15) *N*-(3-cyclopropyl-5-(((3*R*,5*S*)-3,5-dimethylpiperazine-1-yl) methyl)phenyl)-4-(6-methyl-1*H*-indol-3-yl)-5-(trifluoromethyl)pyrimidine-2-amin
   16) (3-(5-chloro-2-((3-cyclopropyl-5-(((3*R*, 5*S*)-3,5-dimethylpiperazine-1-yl)methyl)penyl)amino)pyrimidine-4-yl)-1*H*-indol-6-yl)methanol
   17) 5-chloro-N (3-cyclopropyl-5-(((3*R*, 5*S*)-3,5-dimethylpiperazine-1-yl)methyl)phenyl)-4-(5-methoxy-6-methyl-1*H*-indol-3-yl)pyrimidi ne-2-amin
   18) 3-(5-chloro-2-((3-cyclopropyl-5-(((3*R*, 5*S*)-3,5-dimethylpiperazine-1-yl)methyl)penyl)amino)pyrimidine-4-yl)-6-methyl-1*H*-indol-5-ol
   19) 3-(5-chloro-2-((3-cyclopropyl-5-(((3*R*, 5*S*)-3,5-dimethylpiperazine-1-yl)methyl)penyl)amino)pyrimidine-4-yl)-6-methylindolyn-2-on
   20) 5-chloro-*N*-(3-cyclopropyl-5-(((3*R*, 5*S*)-3,5-dimethylpiperazine-1-yl)methyl)phenyl)-4-methoxy-6-(6-methyl-1*H*-indol-3-yl)pyrimidi ne-2-amin
   21) 5-chloro-2-((3-cyclopropyl-5-(((3*R*, 5*S*)-3,5-dimethylpiperazine-1-yl)methyl)penyl)amino)-6-(6-methyl-1*H*-indol-3-yl)pyrimidine-4-ol
   22) 3-(5-chloro-2-((3-cyclopropyl-5-(((3*R*, 5*S*)-3,5-dimethylpiperazine-1-yl)methyl)penyl)amino)pyrimidine-4-yl)-6-methyl-1*H*-indol-7-ol
   23) 2-((5-chloro-4-(6-methyl-1*H*-indol-3-yl)pyrimidine-2-yl)amino)-4-cyclopropyl-6-(((3*R*, 5*S*)-3,5-dimethylpiperazine-1-yl)methyl)phenol
   24) 4-((5-chloro-4-(6-methyl-1*H*-indol-3-yl)pyrimidine-2-yl)amino)-2-cyclopropyl-6-(((3*R*, 5*S*)-3,5-dimethylpiperazine-1-yl)methyl)phenol
   25) (*R*)-5-chloro-*N*-(3-cyclopropyl-5-((3,3,5-trimethylpiperazine-1-yl)methyl)penyl)-4-(6-methyl-1*H* -indol-3 -yl)pyrimidine-2-amin
   26) ((2*R*, 6*R*)-4-(3-((5-chloro-4-(6-methyl-1*H*-indol-3-yl)pyrimidine-2-yl)amino)-5-cyclopropylbenzyl)-6-methylpiperazine-2-yl)methanol
   27) (*R*)-5-chloro-*N*-(3-cyclopropyl-5-((5-methyl-4,7-diazaspiro[2.5]octane-7-yl)methyl)penyl)-4-(6-methyl-1*H*-indol-3-yl)pyrimidine-2-amin
   28) 5-chloro-*N*-(3-cyclopropyl-5-(((3*R*, 5*R*)-3,5-dimethylpiperazine-1-yl)methyl)phenyl)-4-(6-methyl-1*H*-indol-3-yl)pyrimidine-2-amin
   29) 5-chloro-*N*-(3-cyclopropyl-5-(((3*S*, 5*S*)-3,5-dimethylpiperazine-1-yl)methyl)phenyl)-4-(6-methyl-1*H*-indol-3-yl)pyrimidine-2-amin
   30) 5-chloro-*N*-(3-cyclopropyl-5-(((3*R*, 5*S*)-3,4,5-trimethylpiperazine-1-yl)methyl)penyl)-4-(6-methyl-1*H*-indol-3-yl)pyrimidine-2-amin
   31) (2*R*, 6*S*)-4-(3-((5-chloro-4-(6-methyl-1*H*-indol-3-yl)pyrimidine-2-yl)amino)-5-cyclopropylbenzyl)-2, 6-dimethylpiperazine-1-ol
   32) (2*R*, 6*S*)-4-(3-cyclopropyl-5-((4-(6-methyl-1*H*-indol-3-yl)pyrimidine-2-yl)amino)benzyl)-2,6-dimeth ylpiperazine-1-ol
(7) In the above section (1), a pharmaceutical composition further containing a pharmaceutically acceptable additive.
(8) In the above section (1) or (2), a pharmaceutical composition in which the above cancer is leukemia.
(9) In the above section (8), a pharmaceutical composition in which the said leukemia is acute myelogenous leukemia, acute lymphocytic leukemia or chronic myelogenous leukemia.
(10) In the above section (1) or (2), a pharmaceutical composition for the treatment of cancer by the inhibition of the activity of FLT3 kinase.
(11) In the above section (1) or (2), a pharmaceutical composition for the treatment of cancer with mutation in the tyrosine kinase domain (TKD) of FLT3 amino acid sequence (FLT3-TKD).
(12) In the above section (11), a pharmaceutical composition for the treatment of cancer where the said FLT3-TKD mutation additionally includes internal tandem duplication (ITD) within genes.
(13) In the above section (1) or (2), a pharmaceutical composition for the treatment of cancer with F691L mutation.
(14) In the above section (1) or (2), a pharmaceutical composition for the treatment of cancer with F691L single or ITD-F691L double mutation.
(15) In the above section (14), a pharmaceutical composition where the said cancer is acute myelogenous leukemia (AML,) with F691L single or ITD-F691L double mutation.

In this specification, FLT3 is a member of the class III receptor tyrosine kinase (TK) family usually expressed on the surface of hematopoietic stem cells. FLT3 and its ligand play an important role in the growth, survival and differentiation of pluripotential stem cells. FLT3 is expressed in a number of AML cases. In addition, tyrosine kinase domain (TKD) mutation exists in 28% to 34% and 11% to 14% of AML cases, respectively near D835 in the activated FLT3 and activation loop having internal tandem duplication (ITD) in the juxtamembrane domain and around it. In FLT3, these activating mutations are oncogenic and exhibit deformation activity in cells. Patients with FLT3-ITD mutation have shown bad prognosis in clinical studies and higher recurrence rates and the duration of alleviation from the initial treatment is shorter (6 months compared to 11.5 months of those patients without FLT3-ITD mutation), while their disease-free survival rate and OS have been reduced. Their recurrence rate after a hematopoietic stem cell transplantation (HSCT) is also higher compared to the patients with FLT3-ITD. Similar to the prognosis for the first treatment, patients with recurred and refractory FLT3-mutation-positive AML, have a lower alleviation rate with salvange anticancer therapy, a shorter alleviation period until the second recurrence and a reduced OS compared to FLT3-mutation-negative patients.

In this specification, cancer includes leukemia such as acute myelogenous leukemia (AML,), chronic myelogenous leukemia (CML), acute lymphocytic leukemia (ALL), chronic lymphocytic leukemia (CLL), acute promyelocytic leukemia (APL), hairy cell leukemia, and chronic neutrophilic leukemia (CNL).

In one embodiment, the said cancer can be leukemia.

In one embodiment, the said leukemia can include acute myelogenous leukemia, acute lymphocytic leukemia or chronic myelogenous leukemia.

In this specification, the acute myelogenous leukemia (AML) includes acute myelogenous leukemia having FLT3 mutation. In one embodiment, the acute myelogenous leukemia includes mutant FLT3 polynucleotide-positive acute myelogenous leukemia, FLT3 internal tandem duplication (ITD)- positive acute myelogenous leukemia, or acute myelogenous leukemia having FLT3 point mutation.

The said FLT3 point mutation can be a mutation in the tyrosine kinase domain (TKD) of FLT3 amino acid sequence (FLT3-TKD).

In this specification, "pharmaceutically acceptable" general refers to the components of a non-toxic, inactive and/or physiologically compatible composition or components of a composition. "Pharmaceutical excipient" or "excipient" includes such materials as adjuvant, carrier, pH-modifier and buffering agent, isotonic modifier, humectant and preservative. "Pharmaceutical excipient" is a pharmaceutically acceptable excipient. The said pharmaceutical composition can include a pharmaceutically acceptable typical excipient or additive. The pharmaceutical composition of the present invention can be formulated using a typical method, and it can be manufactured in various oral administration forms such as tablet, pill, powder, capsule, syrup, emulsion, and microemulsion or in parenteral administration forms such as intramuscular, intravenous or subcutaneous administration.

When the said pharmaceutical composition is manufactured in the form of oral formulation, the examples of the carrier or addictive and excipient used include diluent, disintegrant, binder, lubricant, surfactant, suspension or emulsifying agent. When the pharmaceutical composition of the present invention is manufactured in the form of injection, the examples of the said carrier or addictive and excipient include water, saline solution, aqueous glucose solution, aqueous pseudo saccharide solution, alcohol, glycol, ether (e.g. polyethylene glycol 400), oil, fatty acid, fatty acid ester, glyceride, surfactant, suspension or emulsifying agent. This formulation method is widely known to those skilled in the pertinent galenical pharmacy field.

The compound of the chemical formula A or the chemical formula 1 as an active ingredient included in the said pharmaceutical composition can be administered orally or parenterally according to its purpose in a dose valid for the treatment or prevention of an individual or patient, and when it is orally administered, it can be included to ensure that the active ingredient is administered at a daily dose of, for example, 0.01 to 1000 mg, 0.01 to 500 mg, 0.1 to 300 mg or 0.1 to 100 mg per kg of body weight, and when it is administered parenterally, it can be included to ensure that the active ingredient is administered at a daily dose of, for example, 0.01 to 100 mg or 0.1 to 50 mg per kg of body weight, and the said composition can be administered in a single dose or multiple smaller doses. The dose for a particular individual or patient has to be decided in consideration of various relevant factors such as the patient's weight, age, gender, health condition, diet, administration time, method of administration and severity of a disease and understandably, it may be increased or decreased appropriately by a professional, and the above doses are not intended to limit the scope of the present invention in any respect.

Another aspect of the present invention provides a method of preventing or treating cancer using the said pharmaceutical composition.

In one embodiment, the dose, number of administrations or method of administration of the compound used in the said treatment method can vary depending on the object treated, the severity of disease or condition, the speed of administration and the prescribing physician's judgment. Usually, the dose for a person weighing 70 kg is 0.1 to 2,000 mg per day, and for example, it can be administered at a dose of 1 to 1,000 mg or 10 to 2,000 mg. The number of administrations is once or multiple times, and for example, it can be administered once or up to four times or on an on/off schedule, and as for the method of administration, it can be administered through an oral or parenteral route. In some cases, a dose smaller than the range mentioned above may be appropriate, and a higher dose can be used without causing any harmful side effect, while a higher dose can be divided into multiple smaller doses over a day. A physician skilled in the pertinent technical field can easily determine and prescribe the dose of the compound used pro re nata. For example, a physician can start with a dose of the compound of the present invention used in pharmaceutical composition lower than the level required to achieve the intended treatment effect and increase the dose gradually until the intended effect is achieved.

In one embodiment, the said treatment method can use the compound in accordance with one aspect of the present invention solely as active ingredient or in combination with one or more other drugs or pharmaceutical carriers known for the treatment of cancer, tumor or leukemia. A compound selected among the compound of the chemical formula A or chemical formula 1 in accordance with one embodiment, its stereoisomer, tautomer, solvate and pharmaceutically acceptable salt can decrease the activity of FLT3 or strengthen the treatment effect of FLT3 mediated disease when it is administered concurrently with other FLT3 kinase activity inhibiting drugs or other drugs of various mechanism enhancing the efficacy of FLT3 kinase activity inhibition or exhibiting increasing action.

In this specification, the term "treatment" is used as a concept encompassing all of treatment, improvement, amelioration or management. In this specification, the term "treating" or "treatment" refers to inhibiting a disease, e.g. inhibiting disease, condition or impairment in an individual experiencing or exhibiting the pathology or sign of a disease, condition or impairment, preventing an additional occurrence of pathology and/or sign, improving a disease, or reversing pathology and/or sign, e.g. decreasing the severity of a disease.

In this specification, the term "preventing" or "prevention" refers to preventing a disease, e.g. preventing a disease, condition or impairment in an individual who may have a trait of disease, condition or impairment but has not experienced or exhibited the pathology or sign of a disease.

In this specification, the term "individual" or "patient" refers to a certain animal including mammals such as mouse, rat, other rodents, rabbit, dog, cat, pig, cow, lamb, horse or primates and human.

In this specification, such expression as "have", "may have", "include" or "may include" indicates the existence of pertinent characteristics (e.g. value or components such as ingredients) and doesn't rule out the existence of additional characteristics.

### [Effects of the invention]

The pharmaceutical composition in accordance with one aspect of the present invention has an excellent FLT3 inhibit activity, so it can be used effectively for the prevention or treatment of cell proliferating diseases caused by abnormal FLT3 activity of cancer such as leukemia. In addition, the said pharmaceutical composition can be used for the treatment of cancer including leukemia having F691L acquired mutation.

### [Brief Description of the Drawings]

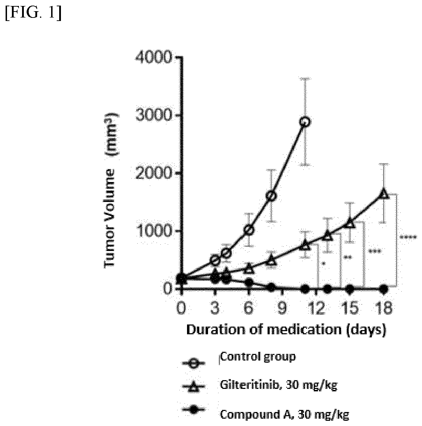

FIG. 1 shows antitumor effects when comparator, compound A and Gilteritinib were administered in nude mice heterografted with MOLM-14 FLT3-ITD/F691L cell strain.

### [Detailed contents to embody the invention]

The present invention is described in detail below.

All technical terms used in the present invention are used in the meanings as generally understood by a person skilled in the art related to the present invention. In addition, desirable methods or samples are described in this specification, but those similar or equivalent to them are also included in the scope of the present invention. In addition, the numbers provided in this specification are considered as having the meaning of "about" even if it is not specified. In this specification, the contents of all publications provided as reference literature are integrated as reference in totality.

The present invention is described below more specifically using the following embodiments and experimental examples. However, these embodiments and experimental examples are just for the facilitation of understanding about the present invention and the scope of the present invention is not limited by them in any sense.

### [Embodiment 1] Kinase inhibition test method

5-chloro-N-(3-cyclopropyl-5-(((3R,5S)-3,5-dimethylpiperazine-1-yl)methyl) phenyl)-4-(6-methyl-1H-indol-3-yl)pyrimidine-2-amin (henceforth referred to as Compound A), which is a FLT3 inhibitor, was measured for the inhibition activity against wild type or mutation type FLT3 and SYK.

Activity measurement was conducted using LanthaScreen test method (wild type and mutation type FLT3) or Z'-LYTE test method (SYK) developed by Thermo Fisher Scientific. LanthaScreen test method is a method of measuring protein activity by measuring fluorescence resonance energy transfer (FRET) signal under the existence of europium-conjugated antibody on the basis of the binding of Alexa Fluor 647-labelled, ATP-competitive kinase inhibitor (kinase tracer-236) to kinase. Z'-LYTE test method is a method using an enzyme which can cut dephosphorylated substrate, and this method measures the activity of kinase protein by measuring two fluorescent FRET signals attached to each end of peptide substrate. The two experiments were all conducted in 384 well plates under the conditions of 50 mM HEPES pH 7.5, 0.01% BRIJ-35, 10 mM MgCl2, 1 mM EGTA, and 1% DMSO. Background signals were measured in the state without each kinase, and after making a measurement by adding only solvent (1% DMSO) as non-inhibiting signals, the Compound A to be assessed was diluted serially into the specified concentration (for example, 50 - 0.05 nM, 1/10 dilution) and the 50% activity inhibition value (IC₅₀) of the Compound A to wild type FLT3, FLT3-ITD, FLT3 D835Y and SYK was calculated using the GraphPad Prism software. Its results are shown in Table 1 below.

**[Table 1]**

| | [Kinase | nM | Kinase | nM |
|---|---|---|---|---|
| FLT3 wild type | | 1.1 | FLT3-ITD | 1.8 |
| SYNC | | 2.9 | FLT3 D835Y | 1.0 |

### [Embodiment 2] Test method of measuring the binding capacity for wild type or mutation type FLT3

The binding capacity of the Compound A for wild type or mutation type FLT3 was measured using the KINOMEscan screening platform of DiscoverX. KINOMEscan test method is a method which measures the binding between the material to be tested and kinase using quantitative PCR by using the competitive binding assay for the active site of the kinase enzyme to which DNA is connected. The test was commissioned to DiscoverX and the binding capacity of the inhibitor for each wild type or mutation type FLT3 was calculated as Kd value. Its results are shown in Table 2 below.

**[Table 2]**

| FLT3 Kinase | oM |
|---|---|
| Wild type | 0.58 |
| ITD | 0.37 |
| D835Y | 0.29 |
| D835V | 0.50 |
| ITD/D835V | 0.48 |
| ITD/F691L | 1.3 |

### [Embodiment 3]

### Mouse model hypodermically implanted with MOLM-14 FLT3-ITD/F691L cell strain

In a mouse model hypodermically implanted with MOLM-14 FLT3-ITD/F691L cell strain, efficacy comparison test was conducted with an FLT3 inhibitor, Compound A, and another FLT3 inhibitor, 6-ethyl-3-[3-methoxy-4-[4-(4-methylpiperazine-1-yl)piperidine-1-yl]anilino]-5-(oxan-4-ylamino )pirazine-2-carboxamide (henceforth referred to as Gilteritinib).

MOLM-14 FLT3-ITD/F691L cell strain was subcutaneously injected into a nude mouse at 3×10^{^6} cell/0. 15mL/mouse and the mouse was left to grow.

The control group was orally administered with DMSO/PEG400/DW (ratio = 0.5/2/7.5, v/v) mixed solution once daily, the Compound A group was orally administered at a dose of 30 mg/kg/day once daily, and the Gilteritinib group was orally administered at a dose of 30 mg/kg/day once daily. The control group was administered for 13 days and the drug administration groups were administered for 18 days with each pertinent drug.

The results of the test are shown in FIG. 1. FIG. 1 shows antitumor effects when comparator, compound A and Gilteritinib were administered in nude mice heterografted with MOLM-14 FLT3-ITD/F691L cell strain. The tumor volume (mm³) of the surviving mice in each group was plotted on the y-axis, whereas the number of days of medication was plotted on the x-axis. In order to evaluate the effects of the administration of drugs, the results of complete response (CR) in which tumor had completely disappeared were checked. As shown in FIG. 1, as a result of measuring and checking the volume of tumor based on the administration of drug, the complete response of the Compound A was shown on Day 12 of administration. In addition, as shown in FIG. 1, the above results showed that the volume of tumor was reduced more significantly in the Compound A administration group than in the Gilteritinib administration group. (^{∗} p <0.05, ^{∗∗} p <0.01, ^{∗∗∗} p <0.001, and ^{∗∗∗∗} p <0.0001 Sidk's test was conducted after two-way analysis of variance)

Until now, the present invention has been examined mainly in terms of its embodiments. Those skilled in the art to which the present invention belongs may understand that the present invention can be embodied in modified forms without departing from the essential characteristics of the present invention, Therefore, the embodiments disclosed above should be considered from an explanatory perspective rather than a restrictive perspective. The scope of the present invention is not shown in the explanation described above but in the scope of patent claims, and all differences within the equivalent scope should be interpreted as being included in the present invention.

## Claims

1. As a pharmaceutical composition comprising an FMS-like tyrosine kinase-3 (FLT3) inhibitor and a pharmaceutically acceptable excipient,
the above FLT3 inhibitor is at least one compound selected from a group comprising a compound of the chemical formula 1 below, its stereoisomer, its tautomer, and their combination, or
is a pharmaceutically acceptable salt of this selected, at least one compound or a solvate of this selected, at least one compound; and
the said pharmaceutical composition is a pharmaceutical composition to treat leukemia, and the said leukemia cancer cells have internal tandem duplication (ITD) variation within FLT3 genes, and
further includes at least one FLT3 mutation selected among F691L, D835Y, D835F, D835I, D835H, D835V and D835A.

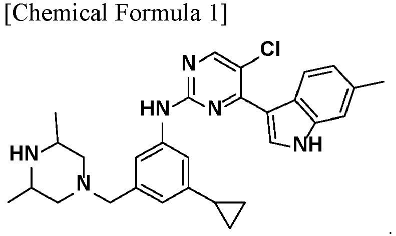

2. A pharmaceutical composition of Claim 1, wherein the said leukemia is acute myeloid leukemia (AML).

3. A pharmaceutical composition of Claim 1, wherein the said pharmaceutical composition is administered concomitantly with an SYK inhibitor simultaneously or sequentially.
